# EUROPÄISCHE PATENTSCHRIFT

(11) **EP 1 296 679 B1**
(45) Veröffentlichungstag und Bekanntmachung des Hinweises auf die Patenterteilung: **15.03.2006**
(21) Anmeldenummer: 01957863.2
(22) Anmeldetag: 19.06.2001
(51) Int. Cl.: A61K 31/415, A61K 31/42, A61K 9/70, A61K 31/18, A61K 31/63, A61K 31/5415

(54) **DERMALES THERAPEUTISCHES SYSTEM ENTHALTEND NICHTSTEROIDALE ANTIPHLOGISTIKA MIT SELEKTIVER COX-2-HEMMUNG**
DERMAL THERAPEUTIC SYSTEM CONTAINING NON-STEROIDAL ANTIPHLOGISTICS WITH SELECTIVE COX-2 INHIBITION
SYSTEME THERAPEUTIQUE DERMIQUE CONTENANT DES ANTIPHLOGISTIQUES NON STEROIDIENS A INHIBITION SELECTIVE DE LA COX-2

(30) Priorität: 01.07.2000 DE 10032132
(43) Veröffentlichungstag der Anmeldung: 02.04.2003
(73) Patentinhaber: LTS LOHMANN Therapie-Systeme AG, 56626 Andernach (DE)
(72) Erfinder: SELZER, Torsten, D-60388 Frankfurt (DE)
(74) Vertreter: Schmidt, Werner
(86) Internationale Anmeldenummer: PCT/EP2001/006905
(87) Internationale Veröffentlichungsnummer: WO 2002/002111

(56) Entgegenhaltungen:
- EP-A- 0 812 587
- EP-A- 1 005 865
- WO-A-01/35883
- WO-A-01/41783
- WO-A-01/52897
- WO-A-96/38418

## Beschreibung

Die Erfindung betrifft ein Dermales Therapeutisches System, das die Abgabe von nichtsteroidalen Antiphlogistika mit selektiver COX-2-Hemmung an die Haut, insbesondere die menschliche Haut ermöglicht.

Dermale Therapeutische Systeme lassen sich einteilen in Transdermale Therapeutische Systeme (TTS) und Superfizielle Therapeutische Systeme (STS).

Transdermale Therapeutische Systeme sind Darreichungsformen, die auf die Haut appliziert werden und die konzipiert sind, einen Arzneistoff systemisch verfügbar zu machen. TTS können den therapeutischen Wert einer Arzneistoffverabreichung erhöhen, indem eine konstante Abgabe des Arzneistoffes über einen längeren Zeitraum in das Blutkompartiment gewährleistet ist. Probleme wie gastrointestinale Intoleranz, niedrige enterale Absorption, "First Pass"-Metabolisierung in der Leber und erhöhte Applikationsfrequenz bei niedrigen Halbwertzeiten können damit umgangen werden.

Superfizielle Therapeutische Systeme (STS) sind Darreichungsformen, die auf die Haut appliziert werden und die konzipiert sind, einen Arzneistoff topisch für die Haut verfügbar zu machen. STS können den therapeutischen Wert einer topischen Arzneistoffverabreichung erhöhen, indem eine konstante Abgabe des Arzneistoffes über einen längeren Zeitraum in das Hautkompartiment gewährleistet ist.

Dermale Therapeutische Systeme bestehen, nach dem Stand der Technik, aus einer arzneistoffundurchlässigen Trägerschicht, einer arzneistoffhaltigen Reservoir- oder Matrixschicht, gegebenenfalls einer Steuermembran sowie einer Haftklebeschicht zur Befestigung auf der Haut, wobei diese mit der arzneistoffhaltigen Schicht identisch sein kann, und einer vor Applikation zu entfernenden, ebenfalls arzneistoffundurchlässigen Schutzschicht. Die arzneistoffhaltige Schicht besteht aus Arzneistoff und Hilfsstoffen, wie z. B. Weichmacher, Klebrigmacher, Lösungsvermittler, Stabilisatoren, Füllstoffe, Trägerstoffe und Permeations-beschleuniger. Die hierfür in Frage kommenden pharmazeutisch unbedenklichen Substanzen sind dem Fachmann bekannt.

Nichtsteroidale Antiphlogistika (NSAR) wie z. B. Diclofenac sind seit längerer Zeit bekannt. Sie werden vielfach oral, parenteral oder auch als Salben zur Behandlung von Krankheiten aus dem rheumatischen Formenkreis, Entzündungen von Gelenken, arthrotischen und degenerativen Gelenkerkrankungen und bei Schmerzen, die mit diesen Erkrankungen in Zusammenhang stehen, verabreicht. Als gefährliche Nebenwirkungen können jedoch, insbesondere bei systemischer Verabreichung u. a. gastrointestinale Blutungen und Perforationen im gastrointestinalen System auftreten. Inzwischen wurde eine neue Stoffklasse von nichtsteroidalen, antiphlogistisch wirkenden Verbindungen entwickelt, welche das Enzym Cyclooxygenase 2 selektiv hemmen und weniger bzw. schwächere Nebenwirkungen zeigen als die oben genannten NSAR. Insbesondere wird das Risiko des Auftretens von gastrointestinalen Komplikationen deutlich herabgesetzt. Zu diesen COX-2-Inhibitoren genannten Verbindungen gehören z. B. Diaryl-2(5H)-furanone und deren Analoga, insbesondere 3-Aryl-4-(4-methyl-sulfonylphenyl)-2-furanone (Chan, C.-C., et al., J. Pharmacol. Exp. Ther. 290 (1999), 551-560), ferner Verbindungen der Klasse 4-[5-Aryl-3-(trifluormethyl)pyrazol-1-yl] benzolsulfonamid (Scarpignato, C. et al., Gastroenterol. Int. 12/4 (1999), 186-215; Bjamason, I., Ital. J. Gastroenterol. Hepatol. 31, Suppl. 1 (1999), 27-36; WO 95/15316).

Rofecoxib (3-Phenyl-4-[4-methylsulfonylphenyl]-2(5H)-furanon) ist das erste auf dem Markt befindliche nichtsteroidale Antiphlogistikum mit selektiver COX-2-Hemmung. Rofecoxib wurde in mehreren klinischen Studien zur symptomatischen Behandlung von arthrotischen Reizzuständen untersucht. In den klinischen Studien waren Perforationen, Ulzera und Blutungen im oberen Gastrointestinaltrakt bei Patienten unter Rofecoxib-Therapie signifikant geringer als bei Patienten unter einer Therapie mit anderen nichtsteroidalen Antirheumatika, die als Vergleichssubstanz dienten. In zwei Endoskopie-Studien über 24 Wochen an Patienten war der Prozentsatz der Patienten mit endoskopisch nachweisbaren gastroduodenalen Ulzera nach 12-wöchiger Behandlungszeit unter Placebo und Rofecoxib 25 bzw. 50 mg/Tag vergleichbar. In beiden Studien war die kumulative Inzidenz gastroduodenaler Ulzera über den Zeitraum von 12 bzw. 24 Wochen unter Rofecoxib signifikant geringer als unter Ibuprofen 2400 mg/Tag.
Rofecoxib ist insbesondere zur Behandlung von Symptomen bei Reizzuständen degenerativer Gelenkerkrankungen (Arthrosen) indiziert. In den USA ist die Substanz auch als Antiphlogistikum zugelassenen.

Die Druckschrift WO-A-96 38418 offenbart Zusammensetzungen (z.B. für parenterale oder transdermale Verabreichung), die COX-2-Inhibitoren enthalten.

Jedoch ist die systemische Verabreichung von Rofecoxib und anderen COX-2-Inhibitatoren ebenfalls mit unerwünschten Nebenwirkungen (wenn auch in schwächerem Maße) verbunden. Als Beispiele sind zu nennen Ödeme, Flüssigkeitseinlagerungen, Bauchschmerzen, Benommenheit, Hypertonie, Sodbrennen, Übelkeit, Erbrechen, Durchfälle, ZNS-Störungen wie Kopfschmerzen, Schwindel, Erbrechen, Tinnitus, Sehstörungen oder Somnolenz. Schwerwiegende Nebenwirkungen wie Nephrotoxizität, Lebertoxizität und gastrointestinale Toxizität können bei längerer Behandlung mit COX-2-Inhibitoren nicht ausgeschlossen werden.

Aufgabe der vorliegenden Erfindung ist es daher, eine Darreichungsform für den COX-2-Inhibitor, insbesondere Rofecoxib bereitzustellen, die den Wirkstoff möglichst selektiv an das zu therapierende, von Arthrose, Entzündungen oder sonstigen rheumatischen Erkrankungen befallene Zielorgane liefert.

Diese Aufgabe wird erfindungsgemäß gelöst durch ein Dermales Therapeutisches System, das derartig konzipiert ist, daß eine konstante Abgabe des Wirkstoffes (COX-2-Inhibitor) über einen längeren Zeitraum an die Haut und die umliegenden Gelenkstrukturen gewährleistet ist, und daß der COX-2-inhibitor zu der Gruppe der 3-Aryl-4-(4-methylsulfonylphenyl)-2(H5)-furanone oder der Gruppe der 4-[5-Aryl-3(trifluoromethylpyrazol-1-yl]benzolsulfonamide gehört.

Die erfindungsgemäßen Systeme können sowohl in Form von Matrixsystemen, als auch in Form von Membran- oder Reservoirsystemen eingesetzt werden. Es ist unerheblich, welche Polymere, Harze und eventuell weitere Zusatzstoffe eingesetzt werden, sofern die Formulierung geeignet ist, den Wirkstoff COX-2-Inhibitor an die Haut abzugeben.
Im einfachsten Fall kann der Wirkstoff in einer Lösung von Grundpolymeren grob, kolloidal oder molekular dispergiert sein, die Mischung auf eine geeignete Unterlage - in der Regel eine silikonisierte thermoplastische Folie - beschichtet werden und, nach Abdampfen der Lösemittelanteile, mit einer weiteren Folie abgedeckt werden, welche die spätere Rückseite des TTS/STS darstellt. Durch Stanzen flächiger Gebilde in der gewünschten geometrischen Form werden TTS/STS aus einem solchen Laminat erhalten.

Geeignete Grundstoffe für das erfindungsgemäße System sind Polymere auf Basis von Acrylsäure oder Methacrylsäure und deren Ester, Isobutylen, EthylenVinylacetat, Kautschuken, Styrol-Diencopolymeren, Synthesekautschuken oder Heißschmelzkleber. Diese Auflistung ist bei weitem nicht vollständig, läßt aber die breite Anwendungsfähigkeit des erfindungsgemäßen Prinzips erkennen.

Die erfindungsgemäßen TTS/STS können weiterhin zwei oder mehrere Matrixschichten aufweisen, wobei die einzelnen Matrixschichten unterschiedliche Konzentrationen an Wirkstoff enthalten können. Ferner können die einzelnen Matrixschichten unterschiedliche Haftkleber aufweisen. Um eine Steuerung der Freisetzung zu ermöglichen, sofern dies nicht durch andere Mechanismen bewirkt wird, kann das Reservoir auch mit einer Steuermembran versehen werden, welche die Abgabe des Wirkstoffs an die Haut steuert.

Der Aufbau des erfindungsgemäßen TTS/STS umfaßt außerdem eine wirkstoffundurchlässige Rückschicht sowie eine ebenfalls wirkstoffundurchlässige Schutzfolie bzw. Abziehfolie. Als Rückschicht eignen sich vor allem Polyester, die sich durch besondere Festigkeit auszeichnen, darüber hinaus aber nahezu beliebige andere hautverträgliche Kunststoffe, wie z. B. Polyvinylchlorid, Ethylenvinylacetat, Vinylacetat, Polyethylen, Polypropylen, Cellulosederivate und viele andere mehr. Im Einzelfall kann die Rückschicht mit einer zusätzlichen Auflage versehen werden, z. B. durch Bedampfung mit Metallen oder anderen diffusionssperrenden Zusatzstoffen wie Siliciumdioxid, Aluminiumoxid oder ähnlichen, dem Fachmann bekannten Stoffe. Für die ablösbare Schutzschicht können dieselben Materialien verwendet werden wie für die Rückschicht, vorausgesetzt, daß sie durch eine geeignete Oberflächenbehandlung wie z. B. Silikonisierung ablösbar sind. Es können aber auch andere ablösbare Schutzschichten wie Polyetrafluorethylen-behandeltes Papier, Cellophan, Polyvinylchlorid oder ähnliche verwendet werden.

Gemäß einer besonderen Ausführungsform kann der Wirkstoff aber auch in einem beutelförmigen Reservoir vorliegen, welches mit einer flüssigen hochviskosen, halbfesten oder thixotropen Matrix gefüllt ist, die den Wirkstoff enthält. Besonders vorteilhaft ist es, wenn das halbfeste oder thixotrope Wirkstoffreservoir einen Gelbildner enthält. Die der Haut abgewandte Beutelrückseite muß dabei wirkstoffundurchlässig sein. Optional kann eine wirkstoffdurchlässige Membran die Steuerung der Wirkstofffreisetzung übernehmen.

Als Hilfsstoffe, die in einer oder mehreren Matrixschichten vorliegen, kommen z. B. Weichmacher in Betracht. In Abhängigkeit von dem verwendeten Polymer können dies sein: höhere Alkohole wie Dodecanol, Undecanol, Octanol, Carbonsäureester wie n-Butyladipat, Triglceride, oder mehrwertige Alkohole.

Falls ein transdermales therapeutisches System (TTS) zur Verabreichung von COX-2-Inhibitatoren verwendet wird, ist in vielen Fällen die Gegenwart eines Permeationsbeschleunigers sinnvoll, wie z. B. Fettalkohole wie Decanol, Dodecanol, Fettsäuren wie Ölsäure, Myristinsäure und Polyoxyethylenfettalkoholether. Vorzugsweise werden von diesen Polyoxylaurylether (Brij®) eingesetzt. Auch Polyoxyethylenfettsäureester und Fettsäureester wie Sorbitanmonolaurat oder Ester von langkettigen Fettsäuren mit Methyl-, Ethyl- oder Isopropylalkohol oder Ester von Fettalkoholen mit Essigsäure oder Milchsäure sowie Stoffe wie Olsäurediethanolamin kommen in Betracht.

### Beispiele

### Beispiel 1: Herstellung eines TTS

Die erfindungsgemäßen TTS können z. B. wie folgt hergestellt werden:
Es werden 50 g Rofecoxib sowie 20 g eines geeigneten permeationsfördernden Stoffes (z. B. Brij®30) in 200 g 1,2-Propandiol gelöst. Diese Lösung wird mittels einer geeigneten Rührapparatur in den Silikonkleber 4301 der Fa. Dow Coming (USA), gegeben und dispergiert, so daß möglichst eine homogene Flüssig-Flüssig-Dispersion entsteht. Diese Dispersion wird mit einer geeigneten Vorrichtung homogen auf eine Trägerfolie, z. B. aus Polyethylenterephthalat beschichtet. Anschließend wird durch eine kontrollierte Trocknung das Lösungsmittel des Silikonklebers sowie etwaige Anteile des Propandiols entfernt. Das so erhaltene Laminat wird anschließend mit einer weiteren Folie aus Polyethylenterephthalat zukaschiert. Zuletzt werden TTS einer bestimmten Fläche ausgestanzt und in ein geeignetes Packmittel verpackt.

### Beispiel 2: Herstellung eines STS

Die erfindungsmäßen STS können z. B. wie folgt hergestellt werden:
Es werden 50 g Rofexoxib in 200 g 1,2-Propandiol gelöst. Diese Lösung wird mittels einer geeigneten Rührapparatur in den Silikonkleber 4301 der Fa. Dow Corning (USA), gegeben und dispergiert, so daß möglichst eine homogene Flüssig-Flüssig-Dispersion entsteht. Diese Dispersion wird mit einer geeigneten Vorrichtung homogen auf eine elastisches Trägergewebe, z. B. aus Polyethylenterephthalat beschichtet. Anschließend wird durch eine kontrollierte Trocknung das Lösungsmittel des Silikonklebers sowie etwaige Anteile des Propandiols entfernt. Das so erhaltene Laminat wird anschießend mit einer weiteren Folie aus Polyethylenterephthalat zukaschiert. Zuletzt werden STS einer bestimmten Fläche ausgestanzt und in ein geeignetes Packmittel verpackt.

## Patentansprüche

1. Dermales therapeutisches System enthaltend als pharmazeutischen Wirkstoff zur Behandlung von Symptomen bei Reizzuständen degenerativer Gelenkerkrankungen und zur Behandlung von postoperativen und Menstruationsschmerzen mindestens einen COX-2-Inhibitor, **dadurch gekennzeichnet, daß** der COX-2-inhibitor zu der Gruppe der 3-Aryl-4-(4-methylsulfonylphenyl)-2 (H5)-furanone oder der Gruppe der 4-[5-Aryl-3(trifluoromethylpyrazol-1-yl] benzolsulfonamide gehört.

2. Dermales therapeutisches System gemäß Anspruch 1, **dadurch gekennzeichnet, daß** der COX-2-Inhibitor 3-Phenyl-4-(4-methylsulfonylphenyl) -2 (H5) -furanon (Rofecoxib) ist.

3. Dermales therapeutisches System gemäß Anspruch 1, **dadurch gekennzeichnet, daß** der COX-2-Inhibitor 4-[5-(5-Methylphenyl)-3-(trifluoromethyl) pyrazol-1-yl] benzolsulfonamid (Celecoxib) ist.

4. Dermales therapeutisches System gemäß einem oder mehreren der vorhergehenden Ansprüche, **dadurch gekennzeichnet, daß** es ein transdermales therapeutisches System (TTS) ist.

5. Dermales therapeutisches System gemäß einem oder mehreren der vorhergehenden Ansprüche 1-8, **dadurch gekennzeichnet, daß** es ein superfizielles therapeutisches System (STS) ist.

6. Dermales therapeutisches System gemäß einem oder mehreren der vorhergehenden Ansprüche, **dadurch gekennzeichnet, daß** die COX-2-Inhibitor-Konzentration im System im Bereich von 0,1 bis 50 Gew.-% liegt.

7. Dermales therapeutisches System gemäß Anspruch 6, **dadurch gekennzeichnet, daß** die COX-2-Inhibitor-Konzentration 1 bis 10 Gew.-% beträgt.

## Revendications

1. Système thérapeutique cutané renfermant en tant que principe actif pharmaceutique pour le traitement de symptômes des états d'irritation de maladies d'articulations dégénératives et pour le traitement de douleurs post-opératoires et de menstruation au moins un inhibiteur de COX-2, **caractérisé en ce que** l'inhibiteur de COX-2 appartient au groupe des 3-aryl-4-[4-méthylsulfonylphényl)-2(H5)-furanones ou au groupe des 4-[5-aryl-3(trifluorométhylpyrazol-1-yl]-benzènesulfonamides.

2. Système thérapeutique cutané selon la revendication 1, **caractérisé en ce que** l'inhibiteur de COX-2 est la 3-phényl-4-[4-méthyl-sulfonylphényl)-2(H5)-furanone (Rofecoxib).

3. Système thérapeutique cutané selon la revendication 1, **caractérisé en ce que** l'inhibiteur de COX-2 est le 4-[5-(5-méthylphényl)-3-(trifluorométhyl)-pyrazol-1-yl]-benzène-sulfonamide (Celecoxib).

4. Système thérapeutique cutané selon une ou plusieurs des revendications précédentes, **caractérisé en ce qu'**il s'agit d'un système thérapeutique transdermique (TTS).

5. Système thérapeutique cutané selon une ou plusieurs des revendications précédentes 1 à 8, **caractérisé en ce qu'**il s'agit d'un système thérapeutique superficiel (STS).

6. Système thérapeutique cutané selon une ou plusieurs des revendications précédentes, **caractérisé en ce que** la concentration en inhibiteur de COX-2 dans le système se trouve dans le domaine de 0,1 à 50 % en masse.

7. Système thérapeutique cutané selon la revendication 6, **caractérisé en ce que** la concentration en inhibiteur de COX-2 est de 1 à 10%

## Claims

1. Dermal therapeutic system comprising as an active pharmaceutical ingredient for the treatment of symptoms associated with irritation from degenerative joint disorders and for the treatment of postoperative and menstrual pain at least one COX-2 inhibitor, **characterized in that** the COX-2 inhibitor belongs to the group of the 3-aryl-4-(4-methylsulphonylphenyl)-2(H5)furanones or the group of 4-[5-aryl-3-(trifluoromethylpyrazol-1-yl]benzenesulphonamides.

2. Dermal therapeutic system according to Claim 1, **characterized in that** the COX-2 inhibitor is 3-phenyl-4-(4-methylsulphonylphenyl)-2(H5)furanone (rofecoxib).

3. Dermal therapeutic system according to Claim 1, **characterized in that** the COX-2 inhibitor is 4-[5-(5-methylphenyl)-3-(trifluoromethyl)pyrazol-1-yl]benzenesulphonamide (celecoxib).

4. Dermal therapeutic system according to one or more of the preceding claims, **characterized in that** it is a transdermal therapeutic system (TTS).

5. Dermal therapeutic system according to one or more of the preceding Claims 1-8, **characterized in that** it is a superficial therapeutic system (STS).

6. Dermal therapeutic system according to one or more of the preceding claims, **characterized in that** the COX-2 inhibitor concentration in the system is in the range from 0.1 to 50% by weight.

7. Dermal therapeutic system according to Claim 6, **characterized in that** the COX-2 inhibitor concentration is from 1 to 10% by weight.
